(19) **Europäisches Patentamt**
**European Patent Office**
**Office européen des brevets**

(11) **EP 2 848 247 A1**

(12) **EUROPEAN PATENT APPLICATION**
published in accordance with Art. 153(4) EPC

(43) Date of publication:
**18.03.2015 Bulletin 2015/12**

(51) Int Cl.:
*A61K 9/48* $^{(2006.01)}$   *A61K 9/16* $^{(2006.01)}$
*A61K 47/30* $^{(2006.01)}$   *A61K 47/48* $^{(2006.01)}$

(21) Application number: **12876106.1**

(86) International application number:
**PCT/KR2012/008412**

(22) Date of filing: **16.10.2012**

(87) International publication number:
**WO 2013/168858 (14.11.2013 Gazette 2013/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.05.2012 KR 20120050197**

(71) Applicant: **Biogenics, Inc.**
**Daejeon 305-510 (KR)**

(72) Inventors:
• **SON, Tae-Hun**
**Daejeon 305-759 (KR)**

• **SHIN, Chan-Jae**
**Daejeon 305-728 (KR)**
• **CHOI, Yong-Han**
**Daejeon 306-788 (KR)**
• **JEONG, Sung-Yoon**
**Gumi-si**
**Gyeongsangbuk-do 730-712 (KR)**
• **GO, Tae-Sung**
**Seoul 152-054 (KR)**

(74) Representative: **Stolmár & Partner**
**Blumenstraße 17**
**80331 München (DE)**

(54) **CAPSULE COMPOSITION CONTAINING INORGANIC NANOPARTICLES FOR UV PROTECTION USING OF HYDROGEL-FORMING POLYMERS, AND METHOD FOR PREPARING SAME**

(57)     The present invention relates to a capsule composition containing UV-blocking inorganic nanoparticles, and a preparation method thereof, in which the UV-blocking inorganic nanoparticles are embedded in hydrophilic polymer capsules using a water-soluble dispersing agent, so that when the composition is applied to the skin, the polymer forms a thin hydrogel film that keeps the inorganic nanoparticles contained in the capsules to prevent the inorganic nanoparticles from penetrating the skin. The method comprises: uniformly mixing UV-blocking inorganic nanoparticles with a water-soluble dispersing agent in water to prepare a dispersion of the inorganic nanoparticles coated with the dispersing agent; adding, to the dispersion, either a hydrophilic polymer capable of forming a hydrogel, or a solution of the hydrophilic polymer in distilled water, to prepare a mixture; and spray-drying the mixture, thereby producing capsule particles containing the dispersing agent-coated UV-blocking inorganic nanoparticles covered by a film formed of the hydrophilic polymer.

FIG. 1

**Description**

BACKGROUND OF THE INVENTION

Field of the Invention

The present invention relates to a capsule composition containing UV-blocking inorganic nanoparticles, and a preparation method thereof, in which the UV-blocking inorganic nanoparticles are embedded in hydrophilic polymer capsules using a water-soluble dispersing agent, so that when the composition is applied to the skin, the polymer can form a thin hydrogel film that can keep the inorganic nanoparticles contained in the capsules, and thus can prevent the inorganic nanoparticles from penetrating the skin.

Description of the Prior Art

Sunlight can be divided according to wavelength into UV light, visible light and infrared light. Among them, UV light has a direct effect on the skin. UV light can be divided into wavelength into UVA (420-320nm), UVB (320-290 nm) and UVC (100-290 nm). In the past, light having a wavelength of 290 nm or less was mostly absorbed by the ozone layer, and thus could not reach the earth's surface. However, in recent years, the ozone layer has been destroyed due to global warming, and for this reason, efforts have been made to prevent skin irritation from being caused by UV light.

As used herein, the term "UV-blocking agents" generally refers to products that functions to protect the skin from the UV light of the sun. UV-blocking agents function to prevent skin cancer, erythema, skin aging phenomena such as discoloration, freckles, dark spots, and wrinkles, and other skin diseases, which are caused by the UV light of the skin. UV blocking agents are classified into those based on chemical action, and those based on physical action, but in recent years, have also been classified into organic UV-blocking agents and inorganic UV-blocking agents. The term "organic UV-blocking agents" refers to products that chemically block UV light by absorbing UV light to reduce the amount of UV light that reaches the skin.

Inorganic UV-blocking agents function to protect the skin by reflecting and scattering UV light, and these include two kinds of inorganic powders, that is, zinc oxide and titanium oxide. It is generally known that the particle size of inorganic powder is inversely proportional to the sun protection factor thereof, and thus the UV protection factor shows a tendency to increase as the particle size of inorganic UV-blocking agents decreases. Thus, in the prior art, it was important to provide the inorganic powder coating technology that modifies the surface of inorganic nanoparticles to improve the touch feeling thereof, and the particle size control-related technology that grind inorganic materials to inorganic nanoparticles that are effectively dispersed in UV-blocking agent formulations.

In recent years, a variety of nanotechnology products have been used in actual life, and the human hazard caused by the *in vivo* physical and chemical reactions of nanomaterials used in such products has been increasingly issued.

To overcome this human hazard problem caused by nanomaterials, products comprising either microparticles, obtained by aggregation of inorganic nanoparticles, or microcapsules obtained by wet methods such as suspension polymerization or coacervation, have been developed. However, microparticles obtained by aggregation of nanoparticles can be crushed or dispersed into nanoparticles during cosmetic production processes in which intense stirring is carried out.

In addition, in conventional wet methods for preparing microcapsules, washing and filtration processes are performed several times to remove residual organic solvents or impurities, and thus a large amount of hazardous wastewater is generated. Thus, a large amount of wastewater is necessarily generated, resulting in increases in energy costs and environmental costs.

Korean Patent No. 10-0744945 discloses hybrid-encapsulated composite particles obtained by coating an inorganic UV-blocking agent with silica sol so as to form 2-20 coating layers. However, Korean Patent No. 10-0744945 still has a problem in that, because the inorganic UV-blocking agent is coated with silica sol alone, the coated silica sol is dispersed during processing while the capsule is disrupted, and thus the inorganic UV-blocking agent is exposed.

Korean Patent No. 10-1062430 owned by the applicant discloses a method for preparing UV-blocking microcapsules containing inorganic nanoparticles, which comprise dissolving and dispersing a water-insoluble polymer, inorganic nanopowder and a natural dispersing agent in an organic solvent, and then evaporating the organic solvent, thereby preparing microcapsule particles. However, this method has a disadvantage in that, because a large amount of the organic solvent is used, the discharge of a large amount of the organic solvent causes environmental problems.

In order to solve the above-described problem, the present inventors have conducted studies to prepare inorganic nanoparticle-containing microparticles under conditions that do not require an organic solvent.

As used herein, the term "hydrogel" refers to a hydrophilic material that, when added to a sufficient amount of water, maintains its three-dimensional shape without being dissolved or dissociated. Hydrogen contains water in an amount corresponding to 10-2,000% of the weight thereof, and requires crosslinks to maintain its three-dimensional shape. Such crosslinks are made by chemical covalent bonding or intermolecular interaction. The former is made from a monomer having 3 or more functional groups, and the latter is formed by hydrophobic bonding, van der Waals bonding, ionic association, crystallites or the like. The latter case has a characteristic in that it changes reversibly from an aqueous solution to a hydrogel due to a change in temperature or pH. Hydrogel is highly biocompatible, and shows a behavior similar to that of biological tissue when it absorbs a large amount of water. Thus, hydrogel has been frequently studied and used in the medical field, the pharmaceutical field, and fields related to the water absorbing property thereof.

The present inventors have designed a method of preparing a capsule composition containing inorganic nanoparticles using a hydrophilic polymer capable of forming a hydrogel in water, without needing an organic solvent, and have found that, when the hydrogel capsules are applied to the skin, the hydrophilic polymer can form a thin hydrogel film that can keep the inorganic nanoparticles contained in the capsules, and thus can prevent the inorganic nanoparticles from penetrating the skin, thereby completing the present invention.

SUMMARY OF THE INVENTION

It is an object of the present invention to provide a capsule composition containing UV-blocking inorganic nanoparticles, and a preparation method thereof, in which the UV-blocking inorganic nanoparticles are embedded in hydrophilic polymer capsules using a water-soluble dispersing agent, so that when the composition is applied to the skin, the polymer can form a thin hydrogel film that can keep the inorganic nanoparticles contained in the capsules, thus can prevent the inorganic nanoparticles from penetrating the skin.

Another object of the present invention is to provide a capsule composition containing UV-blocking inorganic nanoparticles, which enables the inorganic nanoparticles to be applied uniformly to the skin without re-aggregation so that the inherent sun protection factor (SPF) value of the inorganic nanoparticles can be exhibited intact, and a method for preparing the capsule composition.

Still another object of the present invention is to provide a cosmetic formulation containing, as an active ingredient, a hydrogel-forming capsule composition containing UV-blocking inorganic nanoparticles.

To achieve the above objects, the present invention provides a hydrogel-forming capsule composition containing UV-blocking inorganic nanoparticles, the composition comprising: UV-blocking inorganic nanoparticles; a water-soluble dispersing agent serving to form a coating on the UV-blocking inorganic nanoparticles; and a hydrophilic polymer capable of forming a hydrogel in water.

The hydrogel-forming capsule composition containing UV-blocking inorganic nanoparticles the present invention may comprise 30-98.9 wt% of the UV-blocking inorganic nanoparticles, 0.1-30 wt% of the dispersing agent, and 1-40 wt% of the hydrophilic polymer.

Preferably, the UV-blocking inorganic nanoparticles are formed of any one selected from the group consisting of titanium dioxide, zinc oxide, and a mixture thereof; the dispersing agent is any one or a mixture of two or more selected from the group consisting of polyglycerin fatty acid ester, hydrogenated lecithin, inulin lauryl carbamate, and polylysine; and the hydrophilic polymer is any one or a mixture of two or more selected from the group consisting of agar, collagen peptides, hyaluronic acid, starch, modified starches, including hydrolyzed starch and cross-linked starch, dextrin, gamma-PGA, gums, including carrageenan, Arabic gum and xanthan gum, carboxymethylcellulose, gelatin, pectin, alginic acid, and chitosan.

The present invention also provides a method for preparing hydrogel-forming capsules for cosmetic use containing UV-blocking inorganic nanoparticles, the method comprising the steps of: (1) uniformly mixing UV-blocking inorganic nanoparticles with a water-soluble dispersing agent in water to prepare a dispersion of the UV-blocking inorganic nanoparticles coated with the dispersing agent; (2) adding, to the dispersion of step (1), either a hydrophilic polymer capable of forming a hydrogel, or a solution of the hydrophilic polymer in distilled water, to prepare a mixture; and (3) spray-drying the mixture of the step (2), thereby producing capsule particles containing the dispersing agent-coated UV-blocking inorganic nanoparticles covered by a film formed of the hydrophilic polymer.

BRIEF DESCRIPTION OF THE DRAWINGS

FIG. 1 is a flow chart showing a process for preparing hydrogel-forming capsules containing UV-blocking inorganic nanoparticles according to the present invention.

FIG. 2 is a schematic view of hydrogel-forming capsules containing UV-blocking inorganic nanoparticles, prepared in Examples 1 to 40 of the present invention.

FIG. 3 is a scanning electron microscope image of a capsule prepared in Example 8 representative of Examples 1 to 40.

FIG. 4 is a graph showing the results of measuring the size distribution of particles in a dispersion, prepared in Example 8 representative of Examples 1 to 40, using a particle size analyzer.

FIG. 5 is a graph showing the results of measuring the size distribution of particles in capsules, prepared in Example 8 representative of Examples 1 to 40, using a particle size analyzer.

DETAILED DESCRIPTION OF THE INVENTION

The basic concept of the present invention is as follows. UV-blocking inorganic nanoparticles are embedded in hydrophilic polymer capsules using a hydrophilic polymer, which can form a hydrogel in water, and a water-soluble dispersing agent which can increase the dispersibility of the inorganic nanoparticles in the capsules. When the capsules are applied to a cosmetic formulation, hydrogel capsules containing the inorganic nanoparticles will be formed due to water present in the formulation, and when the hydrogel capsules are applied to the skin, the hydrogel polymer will form a thin hydrogel film that can keep the inorganic nanoparticles contained in the capsules, and thus can prevent the inorganic nanoparticles from penetrating the skin. In addition, the UV-blocking inorganic nanoparticles are coated with the water-soluble dispersing agent during the preparation of the capsules, and thus the UV-blocking inorganic nanoparticles can be applied uniformly to the skin without re-aggregation so that the sun protection factor (SPF) value thereof can be exhibited intact.

Hereinafter, a capsule composition containing UV-blocking inorganic nanoparticles according to the present invention will be described in detail.

A hydrogel-forming capsule composition containing UV-blocking inorganic nanoparticles according to the present invention comprises: UV blocking inorganic nanoparticles; a water-soluble dispersing agent capable of increasing the dispersibility of the UV-blocking inorganic nanoparticles in the capsule; and one or more hydrophilic polymers capable of embedding the inorganic nanoparticles in the capsule and of forming a hydrogel in a water system.

The UV-blocking inorganic nanoparticles function to block UV light, and have a size of about 10-30 nm. The UV-blocking inorganic nanoparticles are preferably contained in an amount of 30-98.9 wt% based on the total weight of the composition. If the inorganic nanoparticles are contained in an amount of less than 30 wt%, the effective content of the UV-blocking inorganic agent in the capsules will decrease, and for this reason, a large amount of the capsules should be used in a formulation, making it difficult to ensure the phase stability of the formulation. In addition, the content of the hydrophilic polymer and the dispersing agent in the formulation will increase, and thus when the capsules are applied to a formulation, the hardness of the capsules will be reduced by water so that the capsules can be collapsed. If the UV-blocking inorganic nanoparticles are contained in an amount of more than 98.9 wt%, the content of the hydrophilic polymer and the dispersing agent will relatively decrease, and thus the ability to bind the inorganic nanoparticles will be reduced, so that the hardness of the capsules will decrease. Thus, in this case, when the hydrogel-forming capsules are applied to a formulation, the capsules will be easily collapsed so that the inorganic nanoparticles are separated from the capsules. As such UV-blocking inorganic nanoparticles, titanium dioxide, zinc oxide and the like may be used alone or in combination.

The water-soluble dispersing agent serves to coat the surface of the UV-blocking inorganic nanoparticles so that the nanoparticles are uniformly dispersed in the hydrophilic polymer capsules without aggregation. It may be any one or a mixture of two or more selected from among polyglycerin fatty acid ester, hydrogenated lecithin, inulin lauryl carbamate, and polylysine. The water-soluble dispersing agent is preferably contained in an amount of 0.1-30 wt% based on the total weight of the composition. If the content of the water-soluble dispersing agent in the composition is higher than the upper limit of the above range, the size distribution of the inorganic particles in the capsules will not significantly differ from the size distribution appearing when the content is within the above range, but the collapse potential of the capsules during the preparation of a formulation will increase so that the inorganic nanoparticles can be separated from the capsules. If the content of the water-soluble dispersing agent in the composition is lower than the lower limit of the above range, the ability to coat the inorganic nanoparticles will be insufficient, and thus the uniform dispersion of the inorganic nanoparticles in the capsules cannot be ensured, and when the capsules are applied to the skin, the inorganic nanoparticles will aggregate again, and thus the sun protection factor (SPF) value thereof will significantly decrease.

The hydrophilic polymer capable of forming a hydrogel in water functions to form a coating on the inorganic nanoparticles so as to form microcapsule particles, thus preventing the inorganic nanoparticles from being separated from the capsule particles when the capsule particles are used in a cosmetic formulation. The hydrophilic polymer is preferably contained in an amount of 1-40 wt%, and more preferably 4-15 wt%, based on the total weight of the composition. If the content of the hydrophilic polymer in the composition is less than 1 wt%, it cannot sufficiently cover the UV-blocking inorganic nanoparticles, and thus the capsules will be easily collapsed when they are applied to a formulation, and if the content of the hydrophilic polymer in the composition is more than 30 wt%, the viscosity of the mixture solution will increase rapidly, making it difficult to control the appearance, size and size distribution of particles during the preparation of capsules. Examples of this hydrophilic polymer include agar, collagen peptides, hyaluronic acid, starch, modified starch such as hydrolyzed starch or cross-linked starch, dextrin, gamma-PGA, gums such as carrageenan, Arabic gum or xanthan gum, carboxymethylcellulose, gelatin, pectin, alginic acid, chitosan, and other water-soluble hydrophilic polymers. In addition, one or a mixture of two or more selected from among the above-described polymers may be used.

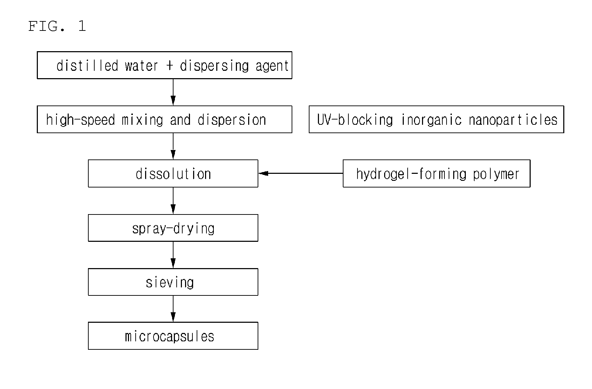

Hereinafter, a method for preparing a hydrogel-forming capsule composition containing UV-blocking inorganic nanoparticles according to the present invention will be described in further detail with reference to FIG. 1.

A method for preparing a hydrogel-forming capsule composition containing UV-blocking inorganic nanoparticles according to the present invention comprises the steps of: (1) uniformly mixing UV-blocking inorganic nanoparticles with a water-soluble dispersing agent in water to prepare a dispersion of the UV-blocking inorganic nanoparticles coated with the dispersing agent; (2) adding, to the dispersion of step (1), either a hydrophilic polymer capable of forming a hydrogel, or a solution of the hydrophilic polymer in distilled water, to prepare a mixture; and (3) spray-drying the mixture, thereby producing capsule particles containing the dispersing agent-coated UV-blocking inorganic nanoparticles covered by a film formed of the hydrogel-forming hydrophilic polymer.

In step (1) of the method of the present invention, the dispersing agent is added to distilled water, and stirred until it is completely dissolved. Then, the inorganic nanoparticles are mixed with the aqueous solution of the dispersing agent so that the surface of the inorganic nanoparticles is coated with the dispersing agent. The mixture is strongly stirred such that the inorganic nanoparticles are dispersed in water so as to have a size of about 100-300 nm, thereby preparing a dispersion of the inorganic nanoparticles coated with the dispersing agent. Herein, the stirring is performed at a temperature of 20~90°C, and preferably 25~70°C.

In step (2), while the aqueous dispersion of the inorganic nanoparticles coated with the dispersing agent in step (1) is stirred at 100-10,000 rpm, preferably 200-3,000 rpm, at a temperature of 20~90°C, preferably 25~70°C, the hydrophilic polymer is slowly added directly to the dispersion, or the hydrophilic polymer completely dissolved in distilled water is added to the dispersion.

In step (3), the mixture formed in step (2) is spraydried, thereby producing microcapsule particles containing the water soluble dispersing agent-coated inorganic nanoparticles covered by a film formed of the hydrophilic polymer.

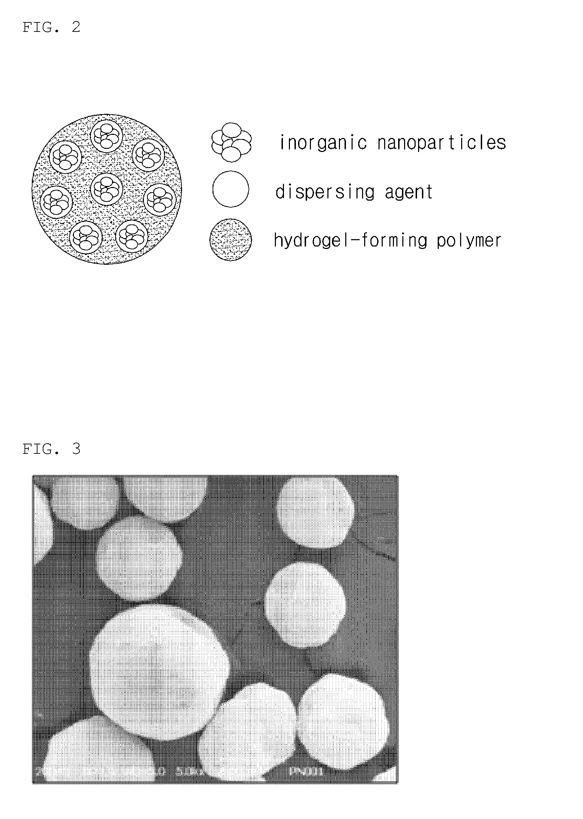

As shown in FIG. 2, the capsule composition prepared according to the above-described method has a shape of spherical capsule particles that have a size of 10-20 $\mu$m and contain the water soluble dispersing agent-coated inorganic nanoparticles dispersed uniformly in the polymer.

According to the present invention, a cosmetic product containing, as a functional ingredient, a hydrogel-forming capsule composition containing UV blocking inorganic nanoparticles, can be prepared according to a conventional method that is used in the field of UV blocking cosmetic products. Because the composition of the present invention is harmless to the human body, it may be formulated in the form of cream, lotion, spray, balm or the like, and may be applied in various ways. If this hydrogel-forming capsule composition containing the UV-blocking inorganic nanoparticles is applied to the skin as a UV blocking agent, it can exhibit the effect of inhibiting skin irritation caused by UV light. The hydrogel-forming capsule composition containing the UV-blocking inorganic nanoparticles according to the present invention may be used as a functional component in a cosmetic composition, and may be contained in an amount of 0.1-20 wt% based on the total weight of the composition.

Hereinafter, the present invention will be described in detail with reference to examples and comparative examples, but these examples are for illustrative purposes only and are not intended to limit the scope of the present invention.

Examples 1 to 40 and Comparative Examples 1 to 8

According to the components and contents shown in Tables 1 to 3 below, hydrogel-forming capsule compositions containing inorganic nanoparticles were prepared. Specifically, a water-soluble dispersing agent was added to 200 g of distilled water, and stirred at 25~70°C until it was completely dissolved. Then, UV-blocking inorganic nanoparticles were added thereto, and the mixture was strongly stirred until inorganic nanoparticles until the inorganic nanoparticles were dispersed to a size of about 100-300 nm, thereby preparing a dispersion of the inorganic nanoparticles coated with the dispersing agent. Then, one or more hydrophilic polymers were added to the dispersion of the water soluble dispersing agent-coated inorganic nanoparticles, and completely dissolved with stirring at 25~70°C and 200-3,000 rpm, thereby preparing a mixture solution. The mixture solution was fed by a metering pump at a constant rate of 80 ml/min into a two-fluid nozzle into which air was being introduced at a pressure of 0.6 MPa and a flow rate of 45 L/min. Herein, the mixture solution was fed into a spray dryer at a temperature of 200°C, and dried in the spray dryer at 90°C, thereby obtaining capsule particles. The obtained capsule particles were sieved through a sieve so as to have an outer diameter of 10-30 $\mu$m.

Table 1 (unit: wt%)

| Composition | | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|---|
| | | | | | | | | | | | | | | | | |
| Inorganic nanoparticles | Titanium dioxide | 93 | 93 | 93 | 93 | 94.5 | 90 | 90 | 85 | 75 | 80 | - | 50 | 42.5 | 35 | 85 |
| | Zinc oxide | - | - | - | - | - | - | - | - | - | - | 85 | 35 | 42.5 | 50 | - |
| Water soluble dispersing agent | Polyglycerin fatty acid ester | 2 | | | | 0.5 | 5 | 5 | 5 | 5 | 10 | 5 | 5 | 5 | 5 | 3 |
| | Hydrogenated lecithin | - | - | - | - | - | - | - | - | - | - | - | - | - | - | - |
| | Inulin lauryl carbamate | - | - | 2 | - | - | - | - | - | - | - | - | - | - | - | 2 |
| | Polylysine | - | | - | 2 | - | | - | - | - | - | - | - | - | - | - |
| -Hydrophilic polymer | Collagen peptide | - | | - | - | - | - | - | - | - | - | - | - | - | - | - |
| | Hyaluronic acid | - | - | - | - | - | - | - | - | - | - | - | - | - | - | - |
| | Corn starch | - | - | 5 | 5 | - | - | - | - | - | 19 | | - | - | - | - |
| | Starch octenyl succinate | | | | | | | | 5 | 10 | 20 | - | 10 | 10 | 10 | 10 |
| | Agar | - | - | - | - | - | - | - | - | - | - | - | - | - | - | - |
| | Carrageenan | - | - | - | - | - | - | - | - | - | - | - | - | - | - | - |
| | Arabic gum | - | - | - | - | - | - | - | - | - | - | - | - | - | - | - |
| | Gelatin | - | - | - | - | - | - | - | - | - | - | - | - | - | - | - |
| | Alginate | - | - | - | - | - | - | - | - | - | - | - | - | - | - | - |
| | Chitosan | - | - | - | - | - | - | - | - | - | - | - | - | - | - | - |

Table 2 (unit: wt%)

| Composition | | Example | | | | | | | | | | | | | | |
|---|---|---|---|---|---|---|---|---|---|---|---|---|---|---|---|---|
| | | 16 | 17 | 18 | 19 | 20 | 21 | 22 | 23 | 24 | 25 | 26 | 27 | 28 | 29 | 30 |
| hiorganic nanoparticles | Titanium dioxide | 85 | 85 | 85 | 95 | 95 | 93 | 93 | 93 | 93 | 93 | 85 | 85 | 91 | 80 | 83 |
| | Zinc oxide | - | - | - | - | - | - | - | - | - | - | - | - | - | - | - |
| Water soluble dispersing agent | Polyglycerin fatty acid ester | 3 | 3 | 5 | 2 | 2 | 2 | 2 | 2 | 2 | 2 | 5 | - | - | - | - |
| | Hydrogenated lecithin | 2 | - | - | - | - | - | - | - | - | - | - | - | - | - | - |
| | Inulin lauryl carbamate | - | - | - | - | - | - | - | - | - | - | - | 5 | 2 | 5 | 5 |
| | Polylysine | - | 2 | - | - | - | - | - | - | - | - | - | - | - | - | - |
| Hydrophilic polymer | Collagen peptide | - | - | 10 | - | - | - | - | - | - | - | 5 | 2 | 5 | - | - |
| | Hyaluronic acid | - | - | - | 3 | - | - | - | - | - | - | - | - | - | - | - |
| | Corn starch | - | - | - | - | - | - | - | - | - | - | 5 | - | - | 5 | - |
| | Starch octenyl succinate | 10 | 10 | - | - | - | - | - | - | - | - | - | 8 | - | 10 | 10 |
| | Agar | - | - | - | - | 3 | - | - | - | - | - | - | - | 2 | - | - |
| | Carrageenan | - | - | - | - | - | 5 | - | - | - | - | - | - | - | - | - |
| | Arabic gum | - | - | - | - | - | - | 5 | - | - | - | - | - | - | - | - |
| | Gelatin | - | - | - | - | - | - | - | 5 | - | - | - | - | - | - | - |
| | Alginate | - | - | - | - | - | - | - | - | 10 | - | - | - | - | - | - |
| | Chitosan | - | - | - | | - | - | - | - | - | 10 | - | - | - | - | - |

Table 3 (unit: wt%)

| Composition | | Examples | | | | | | | | | | Comparative Examples | | |
|---|---|---|---|---|---|---|---|---|---|---|---|---|---|---|
| | | 31 | 32 | 33 | 34 | 35 | 36 | 37 | 38 | 39 | 40 | 1 | 2 | 3 |
| inorganic nanoparticles | Titanium dioxide | 81 | 85 | 85 | 81 | 84 | 42 | 42 | 42 | 42 | 42 | 100 | 95 | 90 |
| | Zinc oxide | - | - | - | - | - | 42 | 42 | 42 | 42 | 42 | - | - | - |
| Water soluble dispersing agent | Polyglycerin fatty acid ester | 5 | 5 | 5 | - | - | 2 | - | 1 | 1 | 2 | - | 5 | - |
| | Hydrogenated lecithin | - | - | - | 2 | - | - | - | 1 | - | - | - | - | - |
| | Inulin lamyl carbamate | - | - | - | 2 | 2 | - | 2 | - | 1 | 2 | - | - | - |
| | Polylysine | - | - | - | - | 2 | - | - | - | - | - | - | - | - |
| Hydrophilic polymer | Collagen peptide | 2 | - | - | 2 | 2 | 2 | 2 | 2 | 2 | 2 | - | - | - |
| | Hyaluronic acid | - | - | - | - | - | - | - | - | - | - | - | - | - |
| | Corn starch | - | - | - | - | - | - | - | - | - | - | - | - | - |
| | Starch octenyl succinate | 12 | - | - | 12 | - | 12 | 12 | 12 | 12 | - | - | - | 10 |
| | Agar | - | - | - | - | - | - | - | - | - | - | - | - | - |
| | Carrageenan | - | - | 5 | 5 | - | - | - | - | - | - | - | - | - |
| | Arabic gum | - | - | - | - | - | - | - | - | - | - | - | - | - |
| | Gelatin | - | - | - | - | - | - | - | - | - | 5 | - | - | - |
| | Alginate | - | 5 | - | - | 5 | - | - | - | - | - | - | - | - |
| | Chitosan | - | - | 5 | - | 5 | - | - | - | - | 5 | - | - | - |

Test Example 1

The capsules prepared in Examples 1 to 40 and Comparative Examples 1 to 3 were measured for capsule sphericity, the size distribution of dispersed particles, the particle size distribution of the capsules, and the collapse resistance of the capsules, and the results of the measurement are shown in Table 4 below.

Measurement of capsule sphericity

To measure the sphericity of the capsule particles prepared in Examples 1 to 40 and Comparative Examples 1 to 3, the capsules were measured with a scanning electron microscope (model: CX-100S, manufactured by COXEM), and the shape of the particles present per 1 mm x 1 mm pixel was measured three times. The measurements were averaged, and the average value was rated on a scale of 1 to 5. The results are shown in Table 4 below. Also, FIG. 3 shows a scanning electron micrograph of the capsule powder prepared in Example 8.

Table 4 shows the results of rating of "capsule sphericity" on a scale of 1 to 5 (1: low capsule sphericity meaning that no capsule was formed; and 5: high capsule sphericity meaning that a capsule close to a spherical shape was formed). Higher sphericity values indicate the formation of capsules closer to a spherical shape.

Table 4

| | Capsule sphericity | | Capsule sphericity | | Capsule sphericity | | Capsule sphericity |
|---|---|---|---|---|---|---|---|
| Example 1 | 4 | Example 12 | 5 | Example 23 | 4 | Example 34 | 5 |
| Example 2 | 4 | Example 13 | 5 | Example 24 | 5 | Example 35 | 5 |
| Example 3 | 4 | Example 14 | 5 | Example 25 | 5 | Example 36 | 5 |
| Example 4 | 5 | Example 15 | 4 | Example 26 | 5 | Example 37 | 5 |
| Example 5 | 4 | Example 16 | 5 | Example 27 | 5 | Example 38 | 5 |
| Example 6 | 4 | Example 17 | 5 | Example 28 | 5 | Example 39 | 5 |
| Example 7 | 4 | Example 18 | 5 | Example 29 | 5 | Example 40 | 4 |
| Example 8 | 5 | Example 19 | 4 | Example 30 | 5 | Comparative Example 1 | 1 |
| Example 9 | 5 | Example 20 | 4 | Example 31 | 5 | Comparative Example 2 | 2 |
| Example 10 | 4 | Example 21 | 4 | Example 32 | 4 | Comparative Example 3 | 3 |
| Example 11 | 5 | Example 22 | 4 | Example 33 | 5 | | |

As can be seen from the results in Table 4 above, the particles prepared in Comparative Examples 1 and 2 had no definite shape and were shapeless, and the capsule compositions prepared in Examples 1 to 40 all showed a capsule sphericity of 4-5, suggesting that good capsules were prepared. Comparative Example 3, in which the hydrophilic polymer was used, showed a sphericity of 3, which was lower than those of the Examples, but higher than those of Comparative Examples 1 and 2.

Measurement of size distribution of dispersed particles

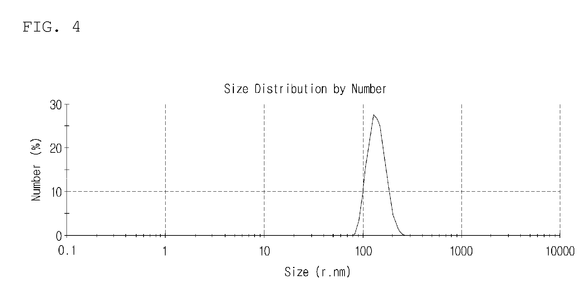

The size of nanoparticles in the dispersion solution produced before the preparation of capsules in Examples 1 to 40 and Comparative Examples 1 to 3 were measured, and the results of the measurement are shown in Table 5 below. Also, FIG. 4 shows the particle size distribution of the dispersion solution for the capsules prepared in Example 8. The size distribution was measured using a Zetasizer Nano-ZS90 system (Malvern). The particle size distribution of nanoparticles was measured three times, and the measurements were averaged. The average value was rated on a scale of 1 to 5 (1: non-uniform dispersion of nanoparticles; and 5: uniform dispersion of nanoparticles). Higher size distribution values indicate that the size distribution of nanoparticles is more uniform and physically stable.

Table 5

| | Size distribution | | Size distribution | | Size distribution | | Size distribution |
|---|---|---|---|---|---|---|---|
| Example 1 | 4 | Example 12 | 5 | Example 23 | 3 | Example 34 | 4 |
| Example 2 | 3 | Example 13 | 5 | Example 24 | 4 | Example 35 | 3 |
| Example 3 | 4 | Example 14 | 5 | Example 25 | 4 | Example 36 | 3 |
| Example 4 | 3 | Example 15 | 4 | Example 26 | 5 | Example 37 | 4 |
| Example 5 | 2 | Example 16 | 4 | Example 27 | 5 | Example 38 | 3 |
| Example 6 | 5 | Example 17 | 5 | Example 28 | 3 | Example 39 | 3 |
| Example 7 | 5 | Example 18 | 5 | Example 29 | 4 | Example 40 | 4 |
| Example 8 | 5 | Example 19 | 4 | Example 30 | 5 | Comparative Example 1 | 1 |
| Example 9 | 4 | Example 20 | 3 | Example 31 | 5 | Comparative Example 2 | 5 |
| Example 10 | 5 | Example 21 | 4 | Example 32 | 4 | Comparative Example 3 | 1 |
| Example 11 | 5 | Example 22 | 3 | Example 33 | 3 | | |

As can be seen from the results in Table 5 above, the dispersions prepared in Examples 1 to 4 and 6 to 40 all showed a high size distribution value of 3 to 5, suggesting that the nanoparticles having a uniform size were embedded in the capsules during the preparation of the capsules. Also, it could be seen that the good size distribution of the nanoparticles in the dispersion solution showed good results in the analysis of UV blocking ability.

However, the size distribution of nanoparticles was poor in the dispersion solutions prepared in Comparative Examples 1 and 3, in which no dispersing agent was used, and Example 5 in which a small amount of the water-soluble dispersing agent was used. In addition, it was observed that a UV blocking agent comprising the capsules prepared in each of Comparative Example 3 and Example 5 had a slightly low sun protection factor (SPF) value.

Measurement of particle size distribution of capsules

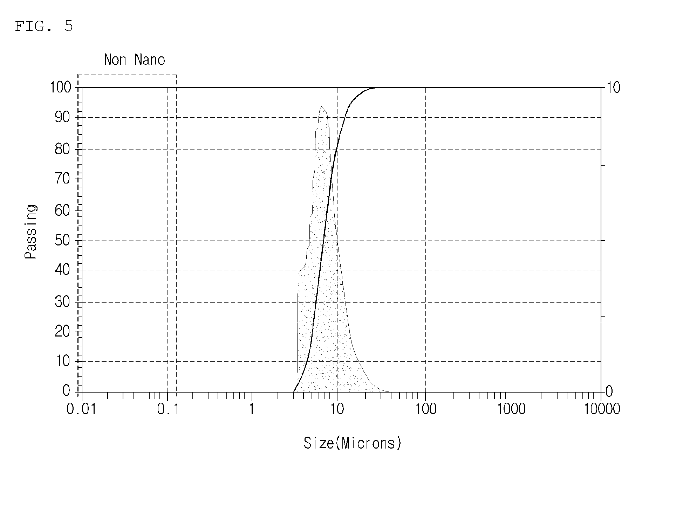

The particle size distribution of the capsules prepared in Examples 1 to 40 and Comparative Examples 1 to 3 was measured, and the results of the measurement are shown in Table 6 below. Also, FIG. 5 shows the particle size distribution of the capsules prepared in Example 1. The particle size distribution was measured with a Microtrac S3500 system (Microtrac Inc.) using isopropyl alcohol as a dispersion medium. The particle size distribution was measured three times, and the measurements were averaged. The average value was rated on a scale of 1 to 5 (1: means that the particle size distribution of the prepared capsules is broad; and 5: means that the particle size distribution of the prepared capsules is narrow). A higher particle size distribution value indicate that the particle size distribution of the prepared capsules is narrower, suggesting that the physical properties of the capsules are more uniform. From the particle size distribution, information on the suitable contents of the components could be seen.

Table 6

|  | Particle size distribution |  | Particle size distribution |  | Particle size distribution |  | Particle size distribution |
|---|---|---|---|---|---|---|---|
| Example 1 | 4 | Example 12 | 5 | Example 23 | 5 | Example 34 | 5 |
| Example 2 | 5 | Example 13 | 5 | Example 24 | 5 | Example 35 | 5 |
| Example 3 | 5 | Example 14 | 5 | Example 25 | 4 | Example 36 | 5 |
| Example 4 | 4 | Example 15 | 4 | Example 26 | 5 | Example 37 | 5 |
| Example 5 | 4 | Example 16 | 5 | Example 27 | 4 | Example 38 | 4 |
| Example 6 | 5 | Example 17 | 5 | Example 28 | 4 | Example 39 | 5 |
| Example 7 | 5 | Example 18 | 5 | Example 29 | 5 | Example 40 | 4 |
| Example 8 | 4 | Example 19 | 5 | Example 30 | 5 | Comparative Example 1 | 1 |
| Example 9 | 5 | Example 20 | 4 | Example 31 | 5 | Comparative Example 2 | 2 |
| Example 10 | 5 | Example 21 | 4 | Example 32 | 4 | Comparative Example 3 | 4 |
| Example 11 | 4 | Example 22 | 5 | Example 33 | 5 |  |  |

As can be seen from the results in Table 6 above, the capsules prepared in Examples 1 to 40 and Comparative Example 3 all had a particle size distribution of 4 to 5, indicating that particles having a relatively uniform size were prepared. However, in Comparative Examples 1 and 2 in which no hydrophilic polymer was used, a very broad particle size distribution was observed. Based on these results, it can be seen that the particle size distribution of the capsules is influenced by whether or not the hydrophilic polymer functioning as the material of the wall of the capsules is used.

Measurement of collapse resistance of capsules

The collapse resistance of the capsule particles prepared in Examples 1 to 40 and Comparative Examples 1 to 3 was measured, and the results of the measurement are shown in Table 7 below. Specifically, 2 g of each of the prepared capsule compositions was added to and dispersed in 200 g of distilled water which was being stirred at 70°C and 4,000 rpm, after which the number-average particle size of the dispersion solution was measured at predetermined time points (10 sec and 10 min) using a particle size analyzer (model: Microtrac S3500, manufactured by Microtrac Inc.). The particle size was measured three times.

```
Collapse  resistance  (%)= Number-average  particle  size
after 10 min of dispersion / number-average particle size after
10 sec of dispersion *100
```

The above-described dispersion conditions are included in the emulsification conditions (50~70°C, and 3,000-4,000 rpm) that are generally applied for cosmetic emulsions, and it is generally known that emulsions are treated for about 3-5 minutes. Thus, it can be seen that a homogenization time of 10 min used in the present invention is a somewhat severe condition.

A higher collapse resistance value (%) of the capsules indicates that the possibility for the capsules to be collapsed during the preparation of cosmetic formulations is lower, and thus the possibility for inorganic nanoparticles to be separated from the capsules is lower. Such capsules can be considered safer against the hazard of nanoparticles.

Table 7

|  | Collapse resistance (%) of capsules |  | Collapse resistance (%) of capsules |  | Collapse resistance (%) of capsules |  | Collapse resistance (%) of capsules |
|---|---|---|---|---|---|---|---|
| Example 1 | 65.3 | Example 12 | 77.5 | Example 23 | 65.9 | Example 34 | 90.7 |
| Example 2 | 63.4 | Example 13 | 74.0 | Example 24 | 81.8 | Example 35 | 88.7 |
| Example 3 | 69.8 | Example 14 | 72.3 | Example 25 | 83.1 | Example 36 | 86.4 |
| Example 4 | 72.1 | Example 15 | 80.8 | Example 26 | 73.4 | Example 37 | 85.1 |
| Example 5 | 78.0 | Example 16 | 71.4 | Example 27 | 75.1 | Example 38 | 93.3 |
| Example 6 | 53.1 | Example 17 | 76.2 | Example 28 | 63.5 | Example 39 | 91.6 |
| Example 7 | 58.6 | Example 18 | 62.3 | Example 29 | 91.5 | Example 40 | 86.4 |
| Example 8 | 80.8 | Example 19 | 51.1 | Example 30 | 90.3 | Comparative Example 1 | 11.7 |
| Example 9 | 75.4 | Example 20 | 53.2 | Example 31 | 91.2 | Comparative Example 2 | 7.5 |
| Example 10 | 57.9 | Example 21 | 73.4 | Example 32 | 81.4 | Comparative Example 3 | 76.9 |
| Example 11 | 75.2 | Example 22 | 58.7 | Example 33 | 85.9 |  |  |

As can be seen from the results in Table 7 above, the capsules prepared in Examples 1 to 40 and Comparative Example 2 all showed a collapse resistance value of 50% or higher under severe conditions (70°C and 4,000 rpm). Also, it was observed that all the particles had a particle size of 2 μm or more even after 10 min of dispersion carried out using strong shear stress, suggesting that the inorganic nanoparticles are securely kept in the capsules by the polymer that forms a hydrogel in water.

However, Comparative Examples 1 and 2 showed a collapse resistance value of 12% or less, and it was observed that some of the particles had a particle size of 200 nm or less after 10 minutes of dispersion. This is because the hydrogel-forming polymer was not used, and thus a binder capable of keeping nanoparticles under dispersion conditions was not present.

Examples 41 to 50 and Comparative Examples 4 and 5

To 95 g of an O/W cream-base composition, a predetermined amount of each of the UV-blocking inorganic nanoparticle-containing capsule compositions prepared in Examples 1, 5, 8, 13, 17, 23, 27 and 34 to 36 was added and mixed, thereby preparing cream formulations. Herein, the capsule composition was added such that the total effective amount of titanium dioxide and zinc oxide in the composition was 5 g. The amounts of components added are shown in Table 8 below. In Comparative Examples 4 and 5, a base formulation containing no UV-blocking inorganic nanoparticles, and a formulation containing 5.88 g of nano titanium dioxide (effective amount of titanium dioxide is 5 g; manufactured by D company), were prepared. The specific components and contents of the O/W cream-based compositions are shown in Table 8 below.

Table 8

| Components | Examples | | | | | | | | | | Comparative Examples | |
|---|---|---|---|---|---|---|---|---|---|---|---|---|
| | 41 | 42 | 43 | 44 | 45 | 46 | 47 | 48 | 49 | 50 | 4 | 5 |
| Polyglyceryl-3 methyl glucose distearate | 2.00 | 2.00 | 2.00 | 2.00 | 2.00 | 2.00 | 2.00 | 2.00 | 2.00 | 2.00 | 2.00 | 2.00 |
| Cetearyl alcohol | 2.00 | 2.00 | 2.00 | 2.00 | 2.00 | 2.00 | 2.00 | 2.00 | 2.00 | 2.00 | 2.00 | 2.00 |
| Glyceryl stearate /PEG-100 stearate | 1.20 | 1.20 | 1.20 | 1.20 | 1.20 | 1.20 | 1.20 | 1.20 | 1.20 | 1.20 | 1.20 | 1.20 |
| Stearic acid | 0.60 | 0.60 | 0.60 | 0.60 | 0.60 | 0.60 | 0.60 | 0.60 | 0.60 | 0.60 | 0.60 | 0.60 |
| Sorbitan stearate | 0.80 | 0.80 | 0.80 | 0.80 | 0.80 | 0.80 | 0.80 | 0.80 | 0.80 | 0.80 | 0.80 | 0.80 |
| Polysorbate 60 | 0.80 | 0.80 | 0.80 | 0.80 | 0.80 | 0.80 | 0.80 | 0.80 | 0.80 | 0.80 | 0.80 | 0.80 |
| Cyclomethicone | 5.50 | 5.50 | 5.50 | 5.50 | 5.50 | 5.50 | 5.50 | 5.50 | 5.50 | 5.50 | 5.50 | 5.50 |
| Dimethicone | 0.30 | 0.30 | 0.30 | 0.30 | 0.30 | 0.30 | 0.30 | 0.30 | 0.30 | 0.30 | 0.30 | 0.30 |
| Ethylhexyl methoxy cinnamate | 7.00 | 7.00 | 7.00 | 7.00 | 7.00 | 7.00 | 7.00 | 7.00 | 7.00 | 7.00 | 7.00 | 7.00 |
| Ethylhexyl salicylate | 2.00 | 2.00 | 2.00 | 2.00 | 2.00 | 2.00 | 2.00 | 2.00 | 2.00 | 2.00 | 2.00 | 2.00 |
| C12-15 alkyl benzoate | 3.00 | 3.00 | 3.00 | 3.00 | 3.00 | 3.00 | 3.00 | 3.00 | 3.00 | 3.00 | 3.00 | 3.00 |
| Chlorphenesin | 0.15 | 0.15 | 0.15 | 0.15 | 0.15 | 0.15 | 0.15 | 0.15 | 0.15 | 0.15 | 0.15 | 0.15 |
| Methyl paraben | 0.15 | 0.15 | 0.15 | 0.15 | 0.15 | 0.15 | 0.15 | 0.15 | 0.15 | 0.15 | 0.15 | 0.15 |
| Butylene glycol | 6.00 | 6.00 | 6.00 | 6.00 | 6.00 | 6.00 | 6.00 | 6.00 | 6.00 | 6.00 | 6.00 | 6.00 |
| Disodium EDTA | 0.05 | 0.05 | 0.05 | 0.05 | 0.05 | 0.05 | 0.05 | 0.05 | 0.05 | 0.05 | 0.05 | 0.05 |
| Xanthan gum | 0.20 | 0.20 | 0.20 | 0.20 | 0.20 | 0.20 | 0.20 | 0.20 | 0.20 | 0.20 | 0.20 | 0.20 |
| Carbomer | 5.00 | 5.00 | 5.00 | 5.00 | 5.00 | 5.00 | 5.00 | 5.00 | 5.00 | 5.00 | 5.00 | 5.00 |
| Magnesium aluminum silicate | 6.00 | 6.00 | 6.00 | 6.00 | 6.00 | 6.00 | 6.00 | 6.00 | 6.00 | 6.00 | 6.00 | 6.00 |
| Water | 51.8 | 51.96 | 51.37 | 51.37 | 51.37 | 51.8 | 51.37 | 51.8 | 51.30 | 51.30 | 62.25 | 51.37 |
| Capsule of Example 1 | 5.37 | - | - | - | - | - | - | - | - | - | - | - |
| Capsule of Example 5 | - | 5.29 | - | - | - | - | - | - | - | - | - | - |
| Capsule of Example 8 | - | - | 5.88 | - | - | - | - | - | - | - | - | - |
| Capsule of Example 13 | - | - | - | 5.88 | - | - | - | - | - | - | - | - |
| Capsule of Example 17 | - | - | - | - | 5.88 | - | - | - | - | - | - | - |

(continued)

| Components | Examples | | | | | | | | | | Comparative Examples | |
|---|---|---|---|---|---|---|---|---|---|---|---|---|
| | 41 | 42 | 43 | 44 | 45 | 46 | 47 | 48 | 49 | 50 | 4 | 5 |
| Capsule of Example 23 | - | - | - | - | - | 5.37 | - | - | - | - | - | - |
| Capsule of Example 27 | - | - | - | - | - | - | 5.88 | - | - | - | - | - |
| Capsule of Example 34 | - | - | - | - | - | - | - | 6.17 | - | - | - | - |
| Capsule of Example 35 | - | - | - | - | - | - | - | - | 5.95 | - | - | - |
| Capsule of Example 36 | - | - | - | - | - | - | - | - | - | 5.95 | - | - |
| Nano titanium dioxide | - | - | - | - | - | - | - | - | - | - | - | 5.88 |
| Total | 100 | 100 | 100 | 100 | 100 | 100 | 100 | 100 | 100 | 100 | 100 | 100 |

Examples 51 to 60 and Comparative Examples 6 and 7

To 95 g of an W/O cream-base composition, a predetermined amount of each of the UV-blocking inorganic nanoparticle-containing capsule compositions prepared in Examples 1, 5, 8, 13, 17, 23, 27 and 34 to 36 was added and mixed, thereby preparing cream formulations. Herein, the capsule composition was added such that the total effective amount of titanium dioxide and zinc oxide in the composition was 5 g. The amounts of components added are shown in Table 8 below. In Comparative Examples 6 and 7, a base formulation containing no UV-blocking inorganic nanoparticles, and a formulation containing 5.88 g of nano titanium dioxide (effective amount of titanium dioxide is 5 g; manufactured by D company), were prepared. The specific components and contents of the W/O cream-based compositions are shown in Table 9 below.

Table 9

| Components | Examples | | | | | | | | | | Comparative Examples | |
|---|---|---|---|---|---|---|---|---|---|---|---|---|
| | 51 | 52 | 53 | 54 | 55 | 56 | 57 | 58 | 59 | 60 | 6 | 7 |
| Phenyl dimethicone | 8.00 | 8.00 | 8.00 | 8.00 | 8.00 | 8.00 | 8.00 | 8.00 | 8.00 | 8.00 | 8.00 | 8.00 |
| Cyclomethicone/dimethicone crosspolymer | 2.00 | 2.00 | 2.00 | 2.00 | 2.00 | 2.00 | 2.00 | 2.00 | 2.00 | 2.00 | 2.00 | 2.00 |
| Cetyl PEG/PPG-10/Dimethicone | 2.00 | 2.00 | 2.00 | 2.00 | 2.00 | 2.00 | 2.00 | 2.00 | 2.00 | 2.00 | 2.00 | 2.00 |
| PEG-10 dimethicone | 0.30 | 0.30 | 0.30 | 0.30 | 0.30 | 0.30 | 0.30 | 0.30 | 0.30 | 0.30 | 0.30 | 0.30 |
| Polyglyceryl-10 dimethicone | 2.00 | 2.00 | 2.00 | 2.00 | 2.00 | 2.00 | 2.00 | 2.00 | 2.00 | 2.00 | 2.00 | 2.00 |
| Stearic acid | 0.30 | 0.30 | 0.30 | 0.30 | 0.30 | 0.30 | 0.30 | 0.30 | 0.30 | 0.30 | 0.30 | 0.30 |
| Trihydroxystearin | 1.50 | 1.50 | 1.50 | 1.50 | 1.50 | 1.50 | 1.50 | 1.50 | 1.50 | 1.50 | 1.50 | 1.50 |
| Ethylhexyl methoxy cinnamate | 7.50 | 7.50 | 7.50 | 7.50 | 7.50 | 7.50 | 7.50 | 7.50 | 7.50 | 7.50 | 7.50 | 7.50 |
| Ethylhexyl salicylate | 2.00 | 2.00 | 2.00 | 2.00 | 2.00 | 2.00 | 2.00 | 2.00 | 2.00 | 2.00 | 2.00 | 2.00 |
| C12-15 alkyl benzoate | 3.00 | 3.00 | 3.00 | 3.00 | 3.00 | 3.00 | 3.00 | 3.00 | 3.00 | 3.00 | 3.00 | 3.00 |
| Chlorphenesin | 0.15 | 0.15 | 0.15 | 0.15 | 0.15 | 0.15 | 0.15 | 0.15 | 0.15 | 0.15 | 0.15 | 0.15 |
| Methyl paraben | 0.15 | 0.15 | 0.15 | 0.15 | 0.15 | 0.15 | 0.15 | 0.15 | 0.15 | 0.15 | 0.15 | 0.15 |
| 2-Octyldodecanol /dibutyl lauroyl glutamide / dibutyl ethylhexanoyl glutamide | 0.80 | 0.80 | 0.80 | 0.80 | 0.80 | 0.80 | 0.80 | 0.80 | 0.80 | 0.80 | 0.80 | 0.80 |
| Glycerin | 4.00 | 4.00 | 4.00 | 4.00 | 4.00 | 4.00 | 4.00 | 4.00 | 4.00 | 4.00 | 4.00 | 4.00 |
| Butylene glycol | 4.00 | 4.00 | 4.00 | 4.00 | 4.00 | 4.00 | 4.00 | 4.00 | 4.00 | 4.00 | 4.00 | 4.00 |
| Magnesium sulfate heptahydrate | 1.00 | 1.00 | 1.00 | 1.00 | 1.00 | 1.00 | 1.00 | 1.00 | 1.00 | 1.00 | 1.00 | 1.00 |
| Disodium EDTA | 0.05 | 0.05 | 0.05 | 0.05 | 0.05 | 0.05 | 0.05 | 0.05 | 0.05 | 0.05 | 0.05 | 0.05 |
| Xanthan gum | 0.05 | 0.05 | 0.05 | 0.05 | 0.05 | 0.05 | 0.05 | 0.05 | 0.05 | 0.05 | 0.05 | 0.05 |
| Water | 55.83 | 55.91 | 55.32 | 55.32 | 55.32 | 55.83 | 55.32 | 55.03 | 55.25 | 55.25 | 61.2 | 55.32 |
| Capsule of Example 1 | 5.37 | - | - | - | - | - | - | - | - | - | - | - |
| Capsule of Example 5 | - | 5.29 | - | - | - | - | - | - | - | | - | - |
| Capsule of Example 8 | - | - | 5.88 | - | - | - | - | - | - | - | - | - |
| Capsule of Example 13 | - | - | - | 5.88 | - | - | - | - | - | - | - | - |
| Capsule of Example 17 | - | - | - | - | 5.88 | - | - | - | - | - | - | - |

EP 2 848 247 A1

(continued)

| Components | Examples | | | | | | | | | | Comparative Examples | |
|---|---|---|---|---|---|---|---|---|---|---|---|---|
| | 51 | 52 | 53 | 54 | 55 | 56 | 57 | 58 | 59 | 60 | 6 | 7 |
| Capsule of Example 23 | - | - | - | - | - | 5.37 | - | - | - | - | - | - |
| Capsule of Example 27 | - | - | - | - | - | - | 5.88 | - | - | - | - | - |
| Capsule of Example 34 | - | - | - | - | - | - | - | 6.17 | - | - | - | - |
| Capsule of Example 35 | - | - | - | - | - | - | - | - | 5.95 | - | - | - |
| Capsule of Example 36 | - | - | - | - | - | - | - | - | - | 5.95 | - | - |
| Nano titanium dioxide | - | - | - | - | - | - | - | - | - | - | - | 5.88 |
| Total | 100 | 100 | 100 | 100 | 100 | 100 | 100 | 100 | 100 | 100 | 100 | 100 |

Test Example 2

The UV blocking effect of each of the cream formulations prepared in Examples 41 to 60 and Comparative Examples 4 to 7 was tested by an in-vitro tester (model: SPF 290S, manufactured by Optometrics). Each sample was scanned 13 times, and analyzed three times, and the measurements are averaged. The results are shown in Table 10 below.

Table 10

| Test groups | Sun protection factor (SPF) | Test groups | Sun protection factor (SPF) |
|---|---|---|---|
| Example 41 (O/W formulation) | 51.4 | Example 51 (W/O formulation) | 49.2 |
| Example 42 (O/W formulation) | 47.6 | Example 52 (W/O formulation) | 46.5 |
| Example 43 (O/W formulation) | 49.1 | Example 53 (W/O formulation) | 48.4 |
| Example 44 (O/W formulation) | 47.6 | Example 54 (W/O formulation) | 51.6 |
| Example 45 (O/W formulation) | 58.1 | Example 55 (W/O formulation) | 55.1 |
| Example 46 (O/W formulation) | 49.8 | Example 56 (W/O formulation) | 47.9 |
| Example 47 (O/W formulation) | 54.7 | Example 57 (W/O formulation) | 50.3 |
| Example 48 (O/W formulation) | 56.6 | Example 58 (W/O formulation) | 52.7 |
| Example 49 (O/W formulation) | 49.8 | Example 59 (W/O formulation) | 53.5 |
| Example 50 (O/W formulation) | 51.1 | Example 60 (W/O formulation) | 48.9 |
| Comparative Example 4 (O/W formulation) | 32.4 | Comparative Example 6 (W/O formulation) | 28.7 |
| Comparative Example 5 (O/W formulation) | 50.5 | Comparative Example 7 (W/O formulation) | 48.2 |

As can be seen from the results in Table 10 above, Comparative Examples 4 and 6, in which no UV-blocking inorganic nanoparticles were used, had low sun protection factor (SPF) values, whereas the sun protection factors of all the Examples were similar to those of Comparative Examples 5 and 7 in which conventional UV blocking powder was used. This indicates that the SPF value of the hydrogel-forming capsules containing the UV blocking inorganic nanoparticles, prepared in the present invention, is similar to that of the conventional UV-blocking nanopowder without being reduced by the polymer film former (hydrogel-forming polymer).

As described above, according to the present invention, hydrogel-forming capsules containing UV-blocking inorganic nanoparticles can be prepared using a dispersing agent and a hydrophilic polymer capable of forming a hydrogel in water. When such capsules are applied to a cosmetic formulation, hydrogel capsules containing the inorganic nanoparticles will be formed due to water present in the formulation, and when these capsules are applied to the skin, the hydrophilic polymer will form a thin hydrogel film that can keep the inorganic nanoparticles contained in the capsules, and thus can prevent the inorganic nanoparticles from penetrating the skin. In addition, because the inorganic nanoparticles are coated with the water-soluble dispersing agent during the preparation of the capsules, the inorganic nanoparticles can be applied uniformly to the skin without re-aggregation, and thus the sun protection factor (SPF) value of the UV-blocking nanoparticles can be exhibited intact. Thus, the UV-blocking inorganic nanoparticle-containing capsules according to the present invention can eliminate concerns about the hazard of the nanoparticles, and enable the SPF value of the inorganic nanoparticles to be exhibited intact.

In addition, according to the present invention, the capsules are prepared by preparing a dispersion of inorganic nanoparticles in water without using an organic solvent such as ethanol, methanol, acetone or the like, followed by spray drying. Thus, no wastewater is generated, environmental problems that can be caused by discharge of an organic solvent can be solved, and energy costs can be reduced.

Moreover, the hydrogel-forming hydrophilic polymers that are used in the present invention are mostly natural materials or natural material derivatives, which do not irritate the human body, and the capsules of the present invention are safer for the human body than other capsules employing synthetic polymers.

Furthermore, when the capsules of the present invention are applied to UV blocking formulations, they will absorb water present in the formulations to form highly flexible hydrogel capsules. Thus, when the capsules of the present invention are applied to the skin, these capsules will give a soft touch feeling without foreign body sensation.

In addition, according to the present invention, the capsules are prepared by preparing a dispersion of inorganic nanoparticles in water without using an organic solvent such as ethanol, methanol, acetone or the like, followed by spray drying. Thus, no wastewater is generated, environmental problems that can be caused by discharge of an organic solvent can be solved, and energy costs can be reduced.

Additionally, using the hydrogel-forming capsules containing the UV-blocking inorganic nanoparticles, prepared as described above, a UV-blocking cosmetic composition that eliminates concerns about the hazard of nanoparticles can be prepared.

**Claims**

1. A hydrogel-forming capsule composition containing UV-blocking inorganic nanoparticles, the composition comprising:

   UV-blocking inorganic nanoparticles;
   a water-soluble dispersing agent serving to form a coating on the UV-blocking inorganic nanoparticles; and
   a hydrophilic polymer capable of forming a hydrogel in water.

2. The hydrogel-forming capsule composition of claim 1, comprising, based on the total weight of the composition, 30-98.9 wt% of the UV-blocking inorganic nanoparticles, 0.1-30 wt% of the water-soluble dispersing agent, and 1-40 wt% of the hydrophilic polymer.

3. The hydrogel-forming capsule composition of claim 1, wherein the UV-blocking inorganic nanoparticles are made of any one selected from the group consisting of titanium dioxide, zinc oxide, and a mixture thereof.

4. The hydrogel-forming capsule composition of claim 1, wherein the water-soluble dispersing agent is any one or more selected from the group consisting of polyglycerin fatty acid ester, hydrogenated lecithin, inulin lauryl carbamate, and polylysine.

5. The hydrogel-forming capsule composition of claim 1, wherein the hydrophilic polymer is any one or more selected from the group consisting of agar, collagen peptides, hyaluronic acid, starch, modified starches, including hydrolyzed starch and cross-linked starch, dextrin, gamma-PGA, gums, including carrageenan, Arabic gum and xanthan gum, carboxymethylcellulose (CMC), gelatin, pectin, alginic acid, and chitosan.

6. A method for preparing hydrogel-forming capsules containing UV-blocking inorganic nanoparticles, the method comprising the steps of:

   (1) uniformly mixing UV-blocking inorganic nanoparticles with a water-soluble dispersing agent in water to prepare a dispersion of the UV-blocking inorganic nanoparticles coated with the dispersing agent;
   (2) adding, to the dispersion of step (1), one or more hydrophilic polymers capable of forming a hydrogel, to prepare a mixture; and
   (3) spray-drying the mixture of step (2), thereby producing capsule particles containing the dispersing agent-coated UV-blocking inorganic nanoparticles covered by a film formed of the hydrophilic polymer.

7. The method of claim 6, wherein the hydrophilic polymer in step (2) is added after dissolution in distilled water.

8. The method of claim 6, wherein the UV-blocking inorganic nanoparticles are made of any one selected from the group consisting of titanium dioxide, zinc oxide, and a mixture thereof.

9. The method of claim 6, wherein the water-soluble dispersing agent is any one or more selected from the group consisting of polyglycerin fatty acid ester, hydrogenated lecithin, inulin lauryl carbamate, and polylysine.

10. The method of claim 6, wherein the hydrophilic polymer is any one or more selected from the group consisting of agar, collagen peptides, hyaluronic acid, starch, modified starches, including hydrolyzed starch and cross-linked starch, dextrin, gamma-PGA, gums, including carrageenan, Arabic gum and xanthan gum, carboxymethylcellulose (CMC), gelatin, pectin, alginic acid, and chitosan.

11. A cosmetic formulation containing, as a functional component, a hydrogel-forming capsule composition containing UV-blocking inorganic nanoparticles according to any one of claims 1 to 5.

FIG. 1

```
┌──────────────────────────────────────┐
│ distilled water + dispersing agent   │
└──────────────────────────────────────┘
                  │
                  ▼
┌──────────────────────────────────────┐     ┌──────────────────────────────────────┐
│ high-speed mixing and dispersion     │     │ UV-blocking inorganic nanoparticles  │
└──────────────────────────────────────┘     └──────────────────────────────────────┘
                  │
                  ▼
┌──────────────────────────┐                  ┌──────────────────────────────┐
│ dissolution              │◄─────────────────│ hydrogel-forming polymer     │
└──────────────────────────┘                  └──────────────────────────────┘
                  │
                  ▼
┌──────────────────────────┐
│ spray-drying             │
└──────────────────────────┘
                  │
                  ▼
┌──────────────────────────┐
│ sieving                  │
└──────────────────────────┘
                  │
                  ▼
┌──────────────────────────┐
│ microcapsules            │
└──────────────────────────┘
```

EP 2 848 247 A1

FIG. 2

inorganic nanoparticles

dispersing agent

hydrogel-forming polymer

FIG. 3

FIG. 4

Size Distribution by Number

FIG. 5

## INTERNATIONAL SEARCH REPORT

| | International application No. |
|---|---|
| | **PCT/KR2012/008412** |

**A. CLASSIFICATION OF SUBJECT MATTER**

*A61K 9/48(2006.01)i, A61K 9/16(2006.01)i, A61K 47/30(2006.01)i, A61K 47/48(2006.01)i*

According to International Patent Classification (IPC) or to both national classification and IPC

**B. FIELDS SEARCHED**

Minimum documentation searched (classification system followed by classification symbols)
A61K 9/48; A61K 8/27; A61Q 19/00; A61K 9/50; A61K 8/11; A61K 8/19; A61K 8/72

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: sunscreen agents, nano inorganic particle, titanium dioxide, nano inorganic particle, water-soluble dispersant, hydrogel, hydrogel, capsule, hydrophilic polymers

**C. DOCUMENTS CONSIDERED TO BE RELEVANT**

| Category* | Citation of document, with indication, where appropriate, of the relevant passages | Relevant to claim No. |
|---|---|---|
| A | KR 10-1062430 B1 (BIOGENICS, INC.) 06 September 2011<br>See the entire document | 1-11 |
| A | KR 10-2009-0069370 A (AMOREPACIFIC CORPORATION) 01 July 2009<br>See the entire document | 1-11 |
| A | KR 10-2001-0049883 A (SHISEIDO COMPANY, LTD.) 15 June 2001<br>See the entire document | 1-11 |
| A | KR 10-2009-0094707 A (NSTECH) 08 September 2009<br>See the entire document | 1-11 |

☐ Further documents are listed in the continuation of Box C. ☒ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 29 MARCH 2013 (29.03.2013) | **01 APRIL 2013 (01.04.2013)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

INTERNATIONAL SEARCH REPORT
Information on patent family members

International application No.

**PCT/KR2012/008412**

| Patent document cited in search report | Publication date | Patent family member | Publication date |
|---|---|---|---|
| KR 10-1062430 B1 | 06.09.2011 | NONE | |
| KR 10-2009-0069370 A | 01.07.2009 | NONE | |
| KR 10-2001-0049883 A | 15.06.2001 | CN 1238106 C0 | 25.01.2006 |
| | | CN 1287877 A0 | 21.03.2001 |
| | | EP 1072259 A2 | 31.01.2001 |
| | | EP 1072259 A3 | 20.03.2002 |
| | | EP 1072259 B1 | 07.06.2006 |
| | | JP 04-637991 B2 | 03.12.2010 |
| | | JP 04-637992 B2 | 03.12.2010 |
| | | JP 04-637993 B2 | 03.12.2010 |
| | | JP 2001-096146 A | 10.04.2001 |
| | | JP 2001-097818 A | 10.04.2001 |
| | | JP 2001-097819 A | 10.04.2001 |
| | | JP 2001-278740 A | 10.10.2001 |
| | | JP 4637991 B2 | 23.02.2011 |
| | | JP 4637992 B2 | 23.02.2011 |
| | | JP 4637993 B2 | 23.02.2011 |
| | | TW I240640B | 01.10.2005 |
| | | TW 240640 A | 01.10.2005 |
| | | TW 240640 B | 01.10.2005 |
| | | US 6391288 B1 | 21.05.2002 |
| KR 10-2009-0094707 A | 08.09.2009 | NONE | |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100744945
- KR 101062430